(19) Europäisches Patentamt
European Patent Office
Office européen des brevets

(11) EP 0 700 398 B1

# (12) EUROPEAN PATENT SPECIFICATION

(45) Date of publication and mention of the grant of the patent:
**26.11.1997 Bulletin 1997/48**

(21) Application number: **94917565.7**

(22) Date of filing: **25.05.1994**

(51) Int Cl.[6]: **C07D 493/04**, A61K 31/37

(86) International application number:
**PCT/DK94/00200**

(87) International publication number:
**WO 94/27998 (08.12.1994 Gazette 1994/27)**

# (54) PSORALEN DERIVATIVES, THEIR PREPARATION AND USE AS MEDICAMENTS

PSORALEN-DERIVATE, DEREN HERSTELLUNG UND VERWENDUNG ALS ARZNEIMITTEL

DERIVES DU PSORALENE, LEUR PREPARATION ET LEUR EMPLOI COMME MEDICAMENTS

(84) Designated Contracting States:
**AT BE CH DE DK ES FR GB GR IE IT LI LU NL PT SE**

(30) Priority: **26.05.1993 DK 607/93**

(43) Date of publication of application:
**13.03.1996 Bulletin 1996/11**

(73) Proprietor: **NOVO NORDISK A/S**
**2880 Bagsvaerd (DK)**

(72) Inventors:
- **HANSEN, John, Bondo**
  **DK-4450 Jyderup (DK)**
- **GR NVALD, Frederik, Christian**
  **DK-2950 Vedb k (DK)**

(56) References cited:

| | |
|---|---|
| EP-A- 0 368 388 | EP-A- 0 377 528 |
| EP-A- 0 402 644 | EP-A- 0 428 437 |
| GB-A- 1 024 385 | US-A- 5 015 740 |

- **CHEMICAL ABSTRACTS, Volume 117, No. 15, 12 October 1992 (12.10.92), (Columbus, Ohio, USA), SETHI, O.P. et al., "Evaluation of Xanthotoxol for Central Nervous System Activity", page 67, The Abstract No. 143317d, J. Ethnopharmacol. 1992, 36 (3), 239-247.**
- **CHEMICAL ABSTRACTS, Volume 110, No. 15, 10 April 1989 (10.04.89), (Columbus, Ohio, USA), HISHMAT, O.H. et al., "Synthesis of Furodiketochroman and Bisfurocoumarin Derivatives and their Biological Activity", page 683, The Abstract No. 134920y, Arch. Pharmacol. Res. 1988, 11 (2), 87-92.**
- **CHEMICAL ABSTRACTS, Volume 107, No. 9, 9 November 1987 (09.11.87), (Columbus, Ohio, USA), JAIN, S.M. et al., "Synthesis of 9Beta-D-Glycopyranosyl Xanthotoxol and CNS Depressant Activity of 9Beta-D-Arabinopyranosyl Xanthotoxol", page 46, The Abstract No. 168615f, Indian J. Pharm. Sci. 1987, 49 (1), 13-16.**
- **CHEMICAL ABSTRACTS, Volume 103, No. 5, 5 August 1985 (05.08.85), (Columbus, Ohio, USA), HANSEN, JOHN B. et al., "Synthesis, Pharmacological Behavior, and DNA Binding of 5-(Aminomethyl)-8-Methoxy-,5-(((3-Aminopropy l)Oxymethyl)-and 8-((3-Aminopropyl)oxy)psoralen Derivatives", page 488, The Abstract No. 37238e, J. Med. Chem. 1985, 28 (8), 1001-1010.**
- **CHEMICAL ABSTRACTS, Volume 92, No. 3, 21 January 1980 (21.01.80), (Columbus, Ohio, USA), SETHI, O.P. et al., "Xanthotoxol (XT)- a Potent 5-HT Antagonist", page 31, The Abstract No. 15291u, Indian J. Physiol. Pharmacol. 1979, 23 (2), 142-143.**
- **CHEMICAL ABSTRACTS, Volume 81, No. 16, 21 October 1974 (21.10.74), (Columbus, Ohio, USA), KOMMISSARENKO, N.F. et al., "Diethylaminoethoxy Psoralen and its Spasmolytic Activity", page 27, The Abstract No. 9768m, Khim. Prir. Soedin. 1974, 10 (1), 88.**
- **J. Med. Chem., Volume 29, 1986, JOSEPH P. YEVICH et al., "Synthesis and Biological Evaluation of 1-(,2-Benzisothiazol-3-yl)-and (1,2-Benzisoxazol-3-yl)piperazine Derivatives as Potential Antipsychotic Agents", page 359 - page 369.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**Description**

The present invention relates to novel compounds which are useful for treating CNS-system, cardiovascular system and/or gastrointestinal disorders, methods for preparing such compounds and pharmaceutical compositions containing them.

Much evidence has accumulated to suggest that neuroleptics exert their antipsychotic action by blocking dopamine (DA) receptors in the brain. In recent years, it has become clear that some neuroleptics (e.g. chlozapine) show an atypical profile: the compounds are not only beneficial in treating patients, who respond poorly to classical neuroleptic therapy, but the compounds are also relatively devoid of extrapyrimidal side effects (EPS) commonly seen with classical neuroleptics (Ereshefsky et al., Clin.Pharm 8, 691-709, 1989). In this respect it has been speculated that atypical neuroleptics are working mainly by blocking so called A10 mesolimbic DA systems (areas which are thought to be affected in psychosis), while the side effects of classical neuroleptics are produced by blockade of DA receptors in the motor areas of the brain (A9 DA system (Gudelsky, Psychopharmacology (Berl) 99: S13-S17, 1989)). The antipsychotic effect of clozapine and related compounds might be due to its blockade of not only DA-receptors (D-1, D-2, D-3, D-4, D-5) but also 5HT-receptor subtypes ($5HT_2$-, $5HT_3$-, $5HT_{1C}$-, $5HT_{1A}$-), NA-$\alpha_1$-receptors, histamine and possibly other receptors.

Furthermore, $5HT_2$-blockade may also be important (Meltzer, Schizphr. Bull. 17: 263-87, 1991) to counteract the so called negative symptoms of psychosis (delusions and social withdrawal) which are otherwise difficult to treat with conventional neuroleptics.

Compounds reducing 5-HT neurotransmission have been suggested to be useful for the treatment of various neurological and psychiatric diseases.

More specifically, the present invention relates to novel compounds of the general formula (I):

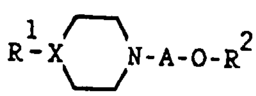

(I)

wherein A is a saturated or unsaturated, straight or branched hydrocarbon containing from 2 to 6 carbon atoms; $R^1$ is

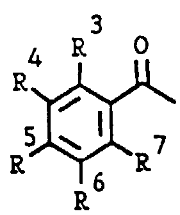

wherein $R^3$, $R^4$, $R^5$, $R^6$ and $R^7$ independently are hydrogen, halogen, $C_{1-6}$-alkyl, $C_{3-8}$-cycloalkyl, cyano, nitro or perhalomethyl; or $R^1$ is

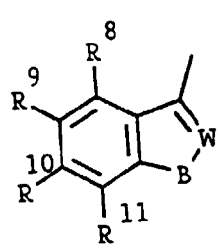

wherein $R^8$, $R^9$, $R^{10}$ and $R^{11}$ independently are hydrogen, halogen, $C_{1-6}$-alkyl, $C_{3-8}$-cycloalkyl, cyano, nitro and perhalomethyl; or

$R^1$ is phenyl, pyridyl, pyrimidyl or pyrazinyl optionally mono- or disubstituted with $C_{1-6}$-alkoxy, halogen, cyano, nitro, acetyl or perhalomethyl, or
$R^1$ is 1-naphthyl, 2-naphthyl, azanaphthyl or diazanaphthyl, either of which may be substituted with $C_{1-6}$-alkyl, $C_{3-8}$-cycloalkyl, $C_{1-6}$-alkoxy, halogen, cyano, nitro, acetyl or perhalomethyl;

B is O, S or NH;

W is N or CH;

X is CH or N;

$R^2$ is a group

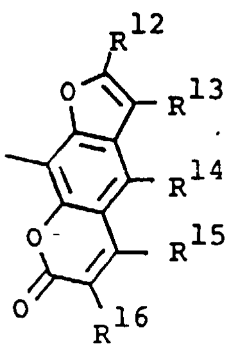
(II)

,

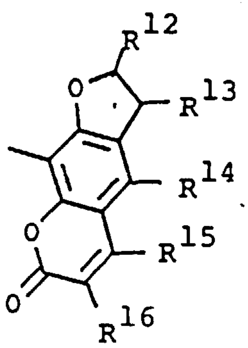
(III)

or

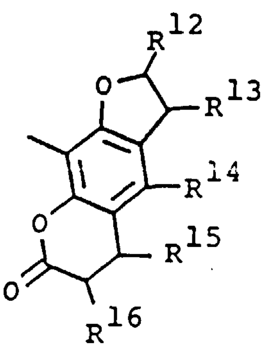
(IV)

wherein $R_{12}$, $R_{13}$, $R_{14}$, $R_{15}$ and $R_{16}$ independently are hydrogen, halogen, $C_{1-6}$-alkyl, cyano, nitro or perhalomethyl.

Physiologically and pharmaceutically acceptable salts of the compounds of the invention include acid addition salts formed with inorganic or organic acids, for example hydrochlorides, hydrobromides, sulphates, nitrates, oxalates, phosphates, tartrates, citrates, fumarates, maleates, succinates, and sulphonates e.g. mesylates. If desirable, selected salts may be subjected to further purification by recrystallization.

The invention includes within its scope all optical isomers of compounds of the general formula I and their mixtures including racemic mixtures thereof.

The term "saturated or unsaturated, straight or branched hydrocarbon containing 2-6 carbon atoms" denotes straight or branched acyclic hydrocarbons of 2-6 carbon atoms having no double or triple bonds or one or more double bonds or one or more triple bonds, e.g. ethyl, propyl, isopropyl, tert. butyl, n-pentyl, n-hexyl, vinyl, allyl, isopropenyl, 2-propynyl, 2-butenyl, 2-pentenyl.

The term "alkoxy" as used herein, alone or in combination, refers to a monovalent substituent comprising a $C_{1-6}$-alkyl group linked through an ether oxygen having its free valence bond from the ether oxygen, e.g. methoxy, ethoxy, propoxy, butoxy, pentoxy.

The term "$C_{1-6}$-alkyl" as used herein, alone or in combination, refers to a straight or branched, saturated hydrocarbon chain having 1-6 carbon atoms such as methyl, ethyl, n-propyl, isopropyl, n-butyl, tert.butyl, n-pentyl, neopentyl, n-hexyl and 2,2-dimethylpropyl.

The term "$C_{3-8}$-cycloalkyl" as used herein denotes a saturated monocyclic hydrocarbon having 3-8 carbon atoms such as cyclopropyl, cyclobutyl, cyclopentyl, cyclohexyl, cyclooctyl.

The term "halogen" means fluorine, chlorine, bromine and iodine, preferably fluorine.

The term "perhalomethyl" means $-CF_3$, $-CCl_3$, $-CBr_3$ and $-CI_3$.

Preferred compounds of the invention are:

9-(3(4-(2-Chlorophenyl)piperazin-1-yl)propoxy)psoralen,
9-(3-(4-(1-Naphthyl)piperazin-1-yl)propoxy)psoralen,
9-(3-(4-(6-Fluoro-1,2-benzisoxazol-3-yl)piperidino)propoxy)psoralen,
9-(3-(4-(2-Pyridylpiperazin-1-yl)propoxy)psoralen,
9-(3-(4-(4-Fluorobenzoyl)piperidino)propoxy)psoralen,

9-(3-(4-(4-Chlorophenyl)piperazin-1-yl)propoxy)psoralen,
9-(3-(4-(4-Acetylphenyl)piperazin-1yl)propoxy)psoralen,
9-(3-(4-(3,4-Dichlorophenyl)piperazin-1yl)propoxy)psoralen,
9-(3-(4-(8-Quinolinyl)piperazin-1-yl)propoxy)psoralen,
9-(4-(4-(4-Fluorobenzoyl)piperidino)butyloxy)psoralen,
9-(4-(4-(6-Fluoro-1,2-benzisoxazol-3-yl)piperidino)butyloxy)psoralen,
9-(4-(4-Fluorobenzoyl)piperidino)butyloxy)-6,7-dihydropsoralen,
9-(2-(4-(4-Fluorobenzoyl)piperidino)ethoxy)psoralen,
9-(2-(4-(6-Fluoro-1,2-benzisoxazol-3-yl)piperidino)ethoxy)psoralen,
9-(2-(4-(1 ,2-Benzisothiazol-3-yl)piperazin-1-yl)ethoxy)psoralen,
5-Chloro-9-(4-(4-(6-fluoro-1,2-benzisoxazol-3-yl)-1-piperidyl)butyloxy)psoralen,
5-Bromo-9-(4-(6-fluoro-1,2-benzisoxazol-3-yl)-1-piperidyl)butyloxy)psoralen,
9-(2-(4-(2-Pyrimidyl)piperazin-1-yl)ethoxy)psoralen,
9-(2-(4-(2-Pyridyl)piperazin-1-yl)-ethoxy)psoralen,
9-(2-(4-(2-Chlorophenyl)piperazin-1-yl)ethoxy)psoralen,
9-(3-[4-(6-Fluoro-1H-indazol-3-yl)piperidin-1-yl]propoxy)psoralen

or pharmaceutically acceptable acid addition salts of these compounds.

The compounds of this invention demonstrate high affinity for various receptor subtypes including the $5HT_2$-, the NA-$\alpha_1$-, the dopamine $D_1$- and $D_2$- receptors or a combination of these.

Accordingly, in another aspect the invention relates to a compound of the general formula (I) or a pharmaceutically acceptable acid addition salt thereof for use as a therapeutically acceptable substance, preferably for use as a therapeutically acceptable substance in the treatment of CNS-system disorders, cardiovascular disorders or gastrointestinal disorders.

Furthermore, the invention also relates to the use of the inventive compounds of formula (I) as medicaments useful for treating CNS-system, cardiovascular system and gastrointestinal disorders, such as treatment of anxiety, sleep disorders, depression, psychosis, schizophrenia, migraine, ischemic neuronal damage, asthma, hypertension, urticaria, analgesia and emesis.

The compound of this invention and physiologically acceptable salts thereof may be prepared by a variety of synthetic routes, which includes reacting a compound of formula (V)

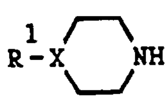

(V)

wherein $R^1$ and X have the meanings set forth above, with a compound of formula (VI)

$$Y\text{-}A\text{-}O\text{-}R^2 \qquad (VI)$$

wherein A and $R^2$ have the meanings set forth above and Y is a suitable leaving group such as halogen, tosylate or mesylate.

A compound of formula VI can be synthesized by a procedure described in C. Antonello et al: Il Farmaco-Ed. Sci. 34, 193 (1979).

Compounds of the formula (I), wherein $R^2$ is either the group (III) or the group (IV) can be prepared by catalytical reduction of compounds of the formula (I), wherein $R^2$ is the group (II), or by reduction of a compound of the formula (VI) wherein $R^2$ is the group (II), or by catalytical reduction of 9-methoxypsoralen, followed by demethylation and reaction with a compound of formula Y-A-Y, wherein Y and A have the meaning set forth above, by a procedure described in C. Antonello et al: Il Farmaco, Ed.Sci. 34, 193 (1979).

In yet another aspect, the invention relates to a method of preparing the compounds of formula (I) as described above.

Compounds of the general formula I were tested for binding to various CNS receptor subtypes.

Detailed conditions for the in vitro assays are described below:

IN-VITRO inhibition of **DOPAMINE D2 receptor binding**.

**Method description**

**Principle:**

Radioactive-labelled ligand $^3$H-Spiroperidol is incubated with isolated cell-membrane fragments at 37°C for a given period of time. Following completed incubation, the incubate is filtered through GF/B filters which are rinsed following filtration to remove unspecifically adhered radioactivity. As opposed to low-molecular compounds, membrane fragments are not rinsed through the filters, the radioactivity bound to the filters is indicative of the amount of ligand bound specifically as well as nonspecifically to the membranes.

**Tissue preparation:**

The procedure is performed in ice bath. Polytron kinematica is rinsed with milli-Q-$H_2O$ before and after use. Male Wistar rats, 150-200 g are decapitated, striatum is removed quickly and weighed (approx. 50 mg). Striatum is transferred to a centrifuging vial containing 10 ml ice-cold D2 buffer. Homogenization is performed applying polytron kinematica (homogenizer) setting 6 for 20 sec. The homogenizer is rinsed with 10 ml D2 buffer in another centrifuging vial. The 10 ml rinsing buffer is added to the tissue vial. Centrifugation at 18,000 rpm for 10 min. at 4°C. Final pellet is transferred to 1,000 x vol. of same buffer. (Ex. 50 mg striatum in 50 ml D2 buffer). Can be stored at 0°C for at least 4 hours. Note that the tissue must be monogeneous (uniform) before use. If not, brief homogenization is performed.

**Assay:**

| | |
|---|---|
| 2,500 µl | tissue (homogeneous) |
| 25 µl | $^3$H-Spiroperidol (0.05 nM) |
| 25 µl | test substance/$H_2O$/blind (Domperidone 0.2 µM) |

Incubation for 20 min. at 37°C - 10 min. on ice bath.

10 ml ice-cold 0.9% NaCl is added to the tubes and filtered through GF/B filters (use gloves). This procedure is repeated. The filters are placed in counting vials and 4 ml opti-flour is added (perform in fume cupboard, use gloves). Counting is performed at window 0-19 of the beta-counter (Pachard). Note that receptor box and lid are rinsed thoroughly in $H_2O$ after use to avoid contamination. Further, the analytical site is cleaned carefully every day after use.

**Test substances:**

Dissolved in $H_2O$, EtOH, MeOH or DMSO and further diluted in $H_2O$. The D2 binding will stand concentrations of up to approx. 20% of these solvents without affecting the binding. Most stock solutions are stable at 4°C, attention is, however, paid to any precipitation, change in colour etc. Test-substance dilutions are always made fresh every day. When weighing out test substances, it is attempted to weigh out approx. 1 mg of substance. Less than 0.8 mg must never be weighed out and only infrequently more than 2 mg (for economy reasons), dependent, however, on conc./assay.

**Results:**

The test value is given as $IC_{50}$ indicating the concentration inhibiting specific binding by 50%.

$$\frac{\text{Conc.}}{\frac{100}{\text{"\% Control"}}-1} = IC_{50}\ (\text{nM})$$

IN-VITRO inhibition of **$Alpha_1$-receptor binding.**

Method description

**Principle:**

Radioactive-labelled ligand $^3$H-Prazosin is incubated with isolated cell-membrane fragments at 25°C for a given period of time. Following completed incubation, the incubate is filtered through GF/B filters, which are rinsed following filtration to remove unspecifically adhered radioactivity. As opposed to low-molecular compounds, membrane fragments are not rinsed through the filters, the radioactivity bound to the filters indicates the amount of ligand bound specifically as well as nonspecifically to the membranes.

**Tissue preparation:**

The procedure is performed in ice bath. Polytron kinematica is rinsed with milli-Q-$H_2O$ before and after use. Male Wistar rats, 150-200 g are decapitated, cortex is removed quickly and weighed (approx. 500 mg). Cortex is transferred to a centrifuging vial containing 10 ml ice-cold D2 buffer. Homogenization applying polytron kinematica (homogenizer) setting 6 for 20 sec. The homogenizer is rinsed with 10 ml D2 buffer in another centrifuging vial. The 10 ml rinsing buffer is added to the tissue vial. Centrifugation at 18,000 rpm for 12 min. at 4°C. This is repeated once. Final pellet is added to 400 x vol. of same buffer. (ex. 500 mg cortex in 200 ml D2 buffer). Can be stored for 30 min. at 0°C.

**Assay:**

| | |
|---|---|
| 2,000 µl | tissue |
| 25 µl | $^3$H-Prazosin (0.5 nM) |
| 25 µl | test substance/$H_2O$/blind Phentolamine (10 µM) |

Incubation for 30 min. at 25°C.

10 ml of ice-cold 0.9% NaCl is added to the tubes and filtered through GF/B filters (use gloves). This procedure is repeated. Filters are placed in counting vials and 4 ml opti-flour is added (perform in fume cupboard, use gloves). Counting is performed at window 0-19 of the beta-counter (Pachard). Note that receptor box and cover are rinsed thoroughly in $H_2O$ after use to avoid contamination. Further, the analytical site is cleaned carefully every day after use.

**Test substances:**

Dissolved in $H_2O$, EtOH, MeOH or DMSO and further diluted in $H_2O$. The binding will stand concentrations of up to approx. 5% of these solvents without affecting the binding. Most stock solutions are stable at 4°C. Attention is, however, paid to any precipitation, change in colour etc. Test-substance dilutions are always made fresh every day. When weighing out test substances, it is attempted to weigh out approx. 1 mg of substance. Less than 0.8 mg must never be weighed out and only infrequently more than 2 mg (for economy reasons), dependent, however, on conc./assay.

**Results:**

The test value is given as $IC_{50}$ indicating the concentration inhibiting specific binding by 50%.

$$\frac{\text{Conc.}}{\frac{100}{\text{"\% Control"}}-1} = IC_{50}\ \text{(nM)}$$

IN-VITRO inhibition of **DOPAMINE D1 receptor binding.**

Method description

**Principle:**

Radioactive-labelled ligand $^3H$-SCH 23390 is incubated with isolated cell-membrane fragments in incubation buffer at 30°C for a given period of time. Following completed incubation, the incubate is filtered through GF/B filters, which are rinsed following filtration to remove unspecifically adhered radioactivity. As opposed to low-molecular compounds, membrane fragments are not rinsed through the filters, the radioactivity bound to the filters indicates the amount of ligand bound specifically as well as nonspecifically to the membranes.

**Tissue preparation:**

Male Wistar rats, 150-200 g are decapitated. Striatum is removed quickly, weighed (approx. 50 mg) and carefully homogenized in 100 x vol. of buffer I applying glass/teflon homogenizer 10 up/down strokes. Ex. 50 mg striatum is homogenized in 5,000 µl buffer I. The homogenate is centrifuged at 18,000 rpm for 20 min. at 4°C, and the supernate is decanted. This step is performed three times, and each time the pellet is resuspended and homogenized in 100 x vol. of buffer I. Following the third centrifugation, the pellet is suspended in 100 x vol. of resuspension buffer and homogenized. The tissue is now ready for use. The tissue is stable at 0°C for 8 hours.

**Assay:**

| | |
|---|---|
| 600 µl | incubation buffer |
| 100 µl | $^3H$-SCH 23390 (0.2 nM) |
| 100 µl | tissue |
| 200 µl | test substance/$H_2O$/blind (cis-flupentixol 2 µM) |

Incubation for 60 min. at 30°C.

10 ml of ice-cold 0.9% NaCl is added to the tubes. Filtration is performed through GF/B filters (use gloves). This procedure is repeated. Filters are placed in counting vials and 4 ml opti-flour is added (perform in fume cupboard, use gloves) and counting is performed at window 0-19 of the beta-counter (Pachard). Note that receptor box and lid are rinsed thoroughly in $H_2O$ after use to avoid contamination. Further, the analytical site is cleaned carefully every day after use.

**Test substances:**

Dissolved in $H_2O$, EtOH, MeOH or DMSO and further diluted in $H_2O$. The D1 binding will stand concentrations of up to approx. 20% of these solvents without affecting the binding. Most stock solutions are stable at 4°C. Attention should, however, be paid to any precipitation, change in colour etc. Test-substance dilutions are always made fresh every day. When weighing out test substances, it is attempted to weigh out approx. 1 mg of substance. Less than 0.8 mg must never be weighed out and only infrequently more than 2 mg (for economy reasons), dependent, however, on conc./assay.

**Results:**

The test value is given as $IC_{50}$ indicating the concentration inhibiting specific binding by 50%.

$$\frac{\text{Conc.}}{\frac{100}{\text{"\% Control"}}-1} = IC_{50}\ (\text{nM})$$

IN VITRO inhibition of **$5HT_2$-receptor binding**

Method description

**Principle:**

Radioactive-labelled ligand $^3$H-Ketanserine is incubated with isolated cell membrane fragments at 37°C for a given period of time. Following completed incubation, the incubate is filtered through GF/B filters, which are rinsed following filtration to remove unspecifically adhered radioactivity. As opposed to low-molecular compounds, membrane fragments are not rinsed through the filters, the radioactivity bound to the filters indicates the amount of ligand bound specifically as well as nonspecifically to the membranes.

**Tissue preparation:**

The preparation is made in ice bath. Polytron kinematica is rinsed with milli-Q-$H_2O$ before and after use. Male Wistar rats, 150-200 g are decapitated. Frontal cortex is removed quickly and weighed (approx. 200 mg). Frontal cortex is added to centrifuging vial containing 10 ml ice-cold D2 buffer. Homogenization applying polytron kinematica (homogenizer) setting 6 for 20 sec. The homogenizer is rinsed with 10 ml D2 buffer in another centrifuging vial. Centrifuged at 18,000 rpm for 10 min. at 4°C. Final pellet is transferred to 125 x vol. of same buffer. (Ex 200 mg in 25 ml D2 buffer). Can be stored for approx. 30 min. at 0°C.

**Assay:**

| | |
|---|---|
| 1250 µl | tissue |
| 25 µl | $^3$H-Ketanserine (0.4 nM) |
| 25 µl | test substance/$H_2O$/blind cyproheptadine (2 µM) |

Incubation for 15 min. at 37°C.

10 ml ice-cold 0.9% NaCl is added to the tubes. Filtration is performed through GF/B filters (use gloves). This procedure is repeated. The filters are placed in counting vials and 4 ml opti-flour is added (prepare in fume cupboard, use gloves). Counting at window 0-19 of the beta-counter (Pachard). Note that receptor box and lid are rinsed thoroughly in $H_2O$ after use to avoid contamination. Further, the analytical site is cleaned carefully every day.

**Test substances:**

Dissolved in $H_2O$, EtOH, MeOH or DMSO and further diluted in $H_2O$. The $5HT_2$ binding will stand concentrations of up to approx. 5% of these solvents without affecting the binding. Most stock solutions are stable at 4°C. Attention should, however, be paid to any precipitation, change in colour etc. Test-substance dilutions are always made fresh every day. When weighing out test substances, it is attempted to weigh out approx. 1 mg of substance. Less than 0.8 mg must never be weighed out and only infrequently more than 2 mg (for economy reasons), dependent, however, on conc./assay.

**Results:**

The test value is given as $IC_{50}$ i.e. the concentration inhibiting specific binding by 50%.

$$\frac{\text{Conc.}}{\frac{100}{\text{"\% Control"}}-1} = IC_{50}\ (\text{nM})$$

The compounds of this invention typically binds to NA-$\alpha_1$, $5HT_2$-, DA-$D_1$-, and DA-$D_2$-receptors, with $IC_{50}$ values in the order of 0.1 nM to 1 µM. Furthermore the compounds are able to antagonize the acetic acid induced writhing in mice with $ED_{50}$-values typically in the order of 0.1 mg/kg to 100 mg/kg.

The compounds of the invention, together with a conventional adjuvant, carrier, or diluent, and if desired a pharmaceutically acceptable acid addition salt thereof, may be placed into the form of pharmaceutical compositions and unit dosages thereof, and in such form may be employed as solids, such as tablets or filled capsules, or liquids, such

as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective central nervous system ailment alleviating amount of the active ingredient commensurate with the intended daily dosage range to be employed. Tablets containing one (1) milligram of active ingredient or, more broadly, one (1) to thirty (30) milligrams, per tablet, are accordingly suitable representative unit dosage forms.

The compounds of this invention can thus be used for the formulation of pharmaceutical preparations, e.g. for oral and parenteral administration to mammals including humans, in accordance with conventional methods of galenic pharmacy.

Conventional excipients are such pharmaceutically acceptable organic or inorganic carrier substances suitable for parenteral or oral application which do not deleteriously react with the active compound.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxiliary agents, such as lubricants, preservatives, stabilizers, wetting agents, emulsifiers, salt for influencing osmotic pressure, buffers and/or colouring substances and the like, which do not deleteriously react with the active compounds.

For parenteral application, particularly suitable are injectable solutions or suspensions, preferably aqueous solutions with the active compound dissolved in polyhydroxylated castor oil.

Ampoules are convenient unit dosage forms.

For oral application, particularly suitable are tablets, dragees, or capsules having talc and/or a carbohydrate carrier or binder or the like, the carrier preferably being lactose and/or corn starch and/or potato starch. A syrup, elixir or like can be used when a sweetened vehicle can be employed. Generally, as to broader ranges, the compound of the invention is dispensed in unit dosage form comprising 0.05-100 mg in a pharmaceutically acceptable carrier per unit dosage.

A typical tablet which may be prepared by conventional tabletting techniques contains:

| | |
|---|---|
| Active compound | 1.0 mg |
| Lactosum | 67.8 mg Ph.Eur. |
| Avicel® | 31.4 mg |
| Amberlite® | 1.0 mg |
| Magnesii stearas | 0.25 mg Ph.Eur. |

The following examples illustrate the specific methods employed in production of a representative number of compounds embraced by this invention.

The following examples illustrate the present invention:

## EXAMPLE 1

### 9-(3-(4-(2-Chlorophenyl)piperazin-1-yl)propoxy)psoralen, HCl

1-(2-Chlorophenyl)piperazin (400 mg; 1.5 mmol), 9-(3-bromopropoxy)psoralen, and potassium carbonate (2.5 g) in acetone (25 ml) was refluxed for 16 h. The mixture was cooled to room temperature, filtered and concentrated in vacuo. The resulting oil was triturated with water and the resulting compound dissolved in ethanol. Addition of concentrated HCl precipitated the desired product as white crystals. Recrystallization from ethanol gave 0.54 g. M.p. 242-243°C. MS (70 eV): m/z 440 ($M^+$, 8%), 438 ($M^+$, 23%), 272 (18), 211 (33), 209 (100), 202 (10).

## EXAMPLE 2

### 9-(3-(4-(1-Naphthyl)piperazin-1-yl)propoxy)psoralen, HCl

Starting from 1-(1-naphthyl)piperazin (0.32 g; 1.4 mmol) and 9-(3-bromopropoxy)psoralen (0.54 g; 1.68 mmol) using the procedure described in example 1 was prepared 0.5 g of the desired product. M.p. > 255°C. MS (70 eV): m/z 454 ($M^+$, 37%), 299 (9), 272 (13), 225 (72), 182 (11), 154 (25), 98 (20), 70 (100).

EXAMPLE 3

9-(3-(4-(6-Fluoro-1,2-benzisoxazol-3-yl)piperidino)propoxy)psoralen, oxalate

Starting from 4-(6-fluoro-1,2-benzisoxazol-3-yl)piperidine, hydrochloride (440 mg, 2.0 mmol), 9-(3-bromopropoxy) psoralen (680 mg, 2.1 mmol) and potassium carbonate (500 mg, 2.1 mmol) using the procedure described in example 1 was prepared 700 mg of the title compound as the free base. This product was dissolved in acetone and oxalic acid (180 mg) in acetone (2 ml) added to precipitate 700 mg desired product as white crystals. M.p. 161-163°C. MS (70 eV): m/z 462 ($M^+$, 42), 324 (28), 233 (50), 190 (16), 122 (10), 96 (100).

EXAMPLE 4

9-(3-(4-(2-Pyridylpiperazin-1-yl)propoxy)psoralen, HCl

Starting from 1-(2-pyridyl)piperazin (250 mg, 1.55 mmol) and 9-(3-bromopropoxy)psoralen (520 mg, 1.6 mmol) using the procedure described in example 1 was prepared 0.54 g of the title compound. M.p. > 230°C. MS (70 eV): m/z 405 ($M^+$, 2%), 311 (10), 298 (8), 286 (22), 202 (5), 185 (15), 107 (83), 70 (100).

EXAMPLE 5

9-(3-(4-(4-Fluorobenzoyl)piperidino)propoxy)psoralen, HCl

4-(4-Fluorobenzoyl)piperidine p-toluene sulfonate (1.15 g, 3 mmol) 9-(3-bromopropoxy)psoralen (1.08 g, 3 mmol) and potassium carbonate (anhyd) (2 g) was stirred in acetone (75 ml) for 10 days at room temperature and at reflux for 3 h. The mixture was conc. in vacuo and then taken up in water/ethyl acetate. The organic phase was dried over $MgSO_4$ and evaporated. The resulting oil was taken up in ethanol and HCl ether added to precipitate a white compound. Recrystallization from ethanol/methanol (3:1) afforded 0.66 g of the title compound. M.p. 237-239° (dec.). MS (70 eV) 449 ($M^+$, 12%), 298 (46), 286 (3), 220 (82), 123 (37), 70 (100).

EXAMPLE 6

9-(3-(4-(4-Chlorophenyl) piperazin-1 -yl) propoxy) psoralen, HCl

Starting from 1-(4-Chlorophenyl)piperazin (0.4 g, 2 mmol) and 9-(3-bromopropoxy)psoralen (0.68 g, 2.1 mmol) using the procedure described in example 1 was prepared 0.75 g of the title compound. M.p. 220-222°C. MS (70 eV): m/z 440 ($M^+$, 10%), 438 ($M^+$, 30%), 298 (7), 286 (3), 272 (19), 237 (2), 211 (32), 209 (98), 70 (100).

EXAMPLE 7

9-(3-(4-(4-Acetylphenyl)piperazin-1-yl)propoxy)psoralen, HCl

Starting from 1-(4-acetylphenyl)piperazin (0.4 g, 2 mmol) and 9-(3-bromopropoxy)psoralen (0.68 g, 2.1 mmol) using the procedure described in example 1 was prepared 0.45 g of the title compound. M.p. 213-215°C. MS (70 eV): m/z 446 ($M^+$, 27%), 298 (8), 286 (3), 272 (13), 217 (100), 174 (15).

EXAMPLE 8

9-(3-(4-(3,4-Dichlorophenyl)piperazin-1-yl)propoxy)psoralen, HCl

Starting from 1-(3,4-dichlorophenyl)piperazin (0.46 g, 2 mmol) and 9-(3-bromopropoxy)psoralen (0.68 g, 2.1 mmol) using the procedure described in example 1 was prepared 0.9 g of the title compound. M.p. 210-212°C. MS (70 eV): m/z 474 ($M^+$, 10%), 472 ($M^+$, 15%), 298 (20), 286 (3), 272 (20), 245 (45), 243 (76), 70 (100).

EXAMPLE 9

9-(3-(4-(8-Quinolinyl)piperazin-1-yl)propoxy)psoralen, HCl

Starting from 1-(8-quinolinyl)piperazin (0.533 g, 2.5 mmol), 9-(3-bromopropoxy)psoralen (0.85 g, 2.63 mmol) and

potassium carbonate (0.414 g, 3 mmol) using the procedure described in example 1 was prepared 0.6 g of the desired product. M.p. 250-251°C. MS (70 eV): m/z 455 ($M^+$, 19%), 298 (12), 254 (3), 170 (27), 157 (63), 129 (35), 70 (100).

EXAMPLE 10

9-(4-(4-(4-Fluorobenzoyl)piperidino)butyloxy)psoralen, oxalate

4-(4-(Fluorobenzoyl)piperidine, p-toluene sulfonate (0.75 g, 2.0 mmol), 9-(4-bromobutyloxy)psoralen (0.71 g, 2.1 mmol) and potassium carbonate (0.6 g, 4.2 mmol) in dry DMF (10 ml) was stirred at 70°C for 16 h and then taken up in water at ethyl acetate. The organic phase was washed with saturated sodium chloride, dried over magnesium sulfate and concentrated in vacuo. The resulting oil was purified by column chromatography (silica gel 60, eluted with ethyl acetate, methanol; 4:1 (v/v)). The product was dissolved in acetone (5 ml) and oxalic acid (150 mg) in 2 ml acetone added to give 320 mg of the title compound. M.p. 115-118°C. MS (70 eV): m/z 463 ($M^+$, 1%), 220 (18), 202 (100), 174 (52).

EXAMPLE 11

9-(4-(4-(6-Fluoro-1,2-benzisoxazol-3-yl)piperidino)butyloxy)psoralen, oxalate

Starting with 6-fluoro-3-(4-piperidinyl)-1,2-benzisoxazol, HCl (450 mg, 2.0 mmol), 9-(4-bromobutyloxy)psoralen (710 mg, 2.1 mmol) and potassium carbonate (600 mg, 4.2 mmol) in 10 ml dry DMF using the procedure described in example 10, 0.36 g of the title compound was prepared. M.p. 127-131°C. MS (70 eV): m/z 476 ($M^+$, 5%), 233 (8%), 202 (100), 174 (72%).

EXAMPLE 12

9-(4-(4-Fluorobenzoyl)piperidino)butyloxy)-6,7-dihydropsoralen, oxalate

9-(4-(4-(6-Fluoro-1,2-benzisoxazol-3-yl)piperidino)butyloxy)psoralen of example 10 (240 mg, 0.52 mmol) was reduced catalytically in 50 ml abs. ethanol (2 atm., room temperature, 50 mg 10% Pd/C). The mixture was filtered and concentrated in vacuo. The product was dissolved in acetone (5 ml) and oxalic acid (25 mg) in 2 ml acetone added to give 42 mg of the title compound. M.p. 114-115°C. MS (70 eV): m/z 465 ($M^+$, 3%), 220 (100).

EXAMPLE 13

9-(2-(4-(4-Fluorobenzoyl)piperidino)ethoxy)psoralen, oxalate

Starting from 4-(4-Fluorobenzoyl)piperidine, p-toluene sulfonate (330 mg, 1.5 mmol), 9-(2-bromoethoxy)psoralen (500 mg, 1.6 mmol) and potassium carbonate (220 mg, 1.6 mmol) using the procedure described in example 10, 300 mg of the title compound was prepared. M.p. 114-115°C. MS (70 eV): m/z 435 ($M^+$, 5%), 284 (23), 234 (3), 220 (100), 202 (1), 123 (43).

EXAMPLE 14

9-(2-(4-(6-Fluoro-1 ,2-benzisoxazol-3-yl)piperidino)ethoxy)psoralen, oxalate

Starting with 4-(6-fluoro-1,2-benzisoxazol-3-yl)piperidine, HCl (450 mg, 2.0 mmol), 9-(2-bromoethoxy)psoralen (400 mg, 1.3 mmol) and potassium carbonate (180 mg, 1.3 mmol) in dry DMF (3 ml) using the procedure described in example 10, 280 mg of the title compound was prepared. M.p. 178-179°C. MS (70 eV) 448 ($M^+$, 29%), 310 (18), 233 (76), 202 (6), 190 (19), 96 (100).

EXAMPLE 15

9-(2-(4-(1,2-Benzisothiazol-3-yl)piperazin-1-yl)ethoxy)psoralen, oxalate

Starting from 1-(1,2-benzisothiazol-1-yl)piperazin (0.25 g, 1 mmol) and 9-(2-bromoethoxy)psoralen (0.36 g, 1.2 mmol) using the procedure described in example 10, 0.2 g of the desired product was prepared. M.p. 157-162°C. MS (70 eV): m/z 447 ($M^+$, 7%), 432 (5), 246 (38), 232 (25), 202 (12), 177 (30), 56 (100).

EXAMPLE 16

5-Chloro-9-(4-(4-(6-fluoro-1,2-benzisoxazol-3-yl)-1-piperidyl)butyloxy)psoralen, HCl

A. To 9-(4-Bromobutyloxy)psoralene (510 mg; 1.5 mmol) in 10 ml dry $CH_2Cl_2$, stirred at 0°C, was added dropwise $Cl_2$ (1.5 ml of a 1.2 M solution in $CCl_4$, 1.8 mM). The solution was stirred for additionally 30 min., washed with $H_2O$ and saturated NaCl, dried over $MgSO_4$ and concentrated in vacuo to give 600 mg 5-chloro-9-(4-bromobutyloxy)psoralene as an oil, which was used in step B without further purification.

MS (70 eV): m/z 372 ($M^+$, 10%), 370 ($M^+$, 85%), 236 (86), 208 (40), 137 (100).

B. 4-(6-Fluoro-1,2-benzisoxazol-3-yl)piperidine, hydrochloride (640 mg, 2.5 mmol), 5-chloro-9-(4-bromobutyloxy) psoralene (550 mg, 1.5 mmol) and $K_2Cl_3$ (420 mg, 3 mmol) in 5 ml dry DMF was stirred at 90°C for 1.5 h. The mixture was cooled to room temperature and taken up in water and ether.

The organic phase was washed with water and saturated NaCl, dried over $MgSO_4$ and concentrated in vacuo to give 650 mg oil. The product was purified by column chromatography (silica gel 60, eluted with methylene chloride, methanol, 9:1 (v/v)). The resulting oil was crystallized by trituration with acetone and subsequently dissolved in 1 ml absolute ethanol. Addition of HCl in ether precipitated 350 mg of the title compound as white crystals. M.p. 138-140°C. MS (FAB): 511 ($M^+$ 1).

EXAMPLE 17

5-Bromo-9-(4-(6-fluoro-1,2-benzisoxazol-3-yl)-1-piperidyl)butyloxy)psoralen, HCl

A. To 9-(4-bromobutyloxy)psoralene (850 mg, 2.5 mmol) in 5 ml glacial acetic acid was added slowly $Br_2$ (480 mg, 3 mmol) dissolved in 3 ml glacial acetic acid. The mixturewas stirred at room temperature for 1 h, neutralized by addition of 4N NaOH and extracted with ethyl acetate. The organic phase was washed with water and saturated NaCl, dried over $MgSO_4$ and concentrated in vacuo. The resulting crystals was washed with ether and ethyl acetate and dried to give 0.5 g of 5-bromo-9-(4-bromobutyloxy)psoralene. M.p. 101-102°C.

B. Starting from 4-(6-fluoro-1,2-benzisoxazol-3-yl)piperidine, hydrochloride (1.0 g, 4 mmol) and 5-bromo-9-(4-bromobutyloxy)psoralene (420 mg, 1 mmol) and potassium carbonate (140 mg, 1 mmol) using the procedure described in example 16 was prepared 370 mg of the title compound as white crystals. M.p. 181-182°C. MS (FAB): 255 (M+1).

EXAMPLE 18

9-(2-(4-(2-Pyrimidyl)piperazin-1-yl)ethoxy)psoralen, oxalate

Starting with 1-(2-pyrimidyl)piperazin, dihydrochloride (470 mg, 2.0 mmol), 9-(2-bromoethoxy)psoralene (310 mg, 1 mmol) and potassium carbonate (410 mg, 3 mmol) using the procedure described in example 10 was prepared 300 mg of the title compound as white crystals. M.p. 199-200°C. MS (70 eV): m/z 392 ($M^+$, 18%), 284 (19), 272 (13), 191 (48), 177 (100).

EXAMPLE 19

9-(2-(4-(2-Pyridyl)piperazin-1-yl)-ethoxy)psoralen, HCl

Starting with 1-(2-pyridyl)piperazine (250 mg, 1.5 mmol), 9-(2-bromoethoxy)psoralene (310 mg, 1 mmol) and potassium carbonate (410 mg, 3 mmol) in dry DMF using the procedure described in example 16 was prepared 250 mg of the title compound as white crystals. M.p. 213-213°C.

MS (70 eV): 391 ($M^+$, 3%), 284 (10), 272 (7), 228 (18), 190 (100).

EXAMPLE 20

9-(2-(4-(2-Chlorophenyl)piperazin-1-yl)ethoxy)psoralen, hydrochloride

Starting with 9-(2-bromoethoxy)psoralene (0.3 g, 1 mmol), 1-(2-chlorophenyl)piperazine, hydrochloride and potassium carbonate in 50 ml acetone, using the procedure described in example 1 was prepared 140 mg of the title compound as white crystals. M.p. 137-138°C.

NMR (400 MHz, DMSO-$d_6$) δ (ppm): 3.3-3.5 (m, 6H); 3.7-3.8 (m, 4H); 4.85 (bs, 2H); 6.5 (d, J=9Hz, 1H); 7.10-7.5 (t, J=8Hz, 1H); 7.16 (bs, 1H); 7.25 (d, J=8Hz, 1H); 7.35 (t, J=8Hz, 1H); 7.46 (d, J=8Hz, 1H); 7.8 (s, 1H); 8.16 (s, 1H);

8.2 (d, J=9Hz, 1H); 11.25 (bs, 1H).

EXAMPLE 21

9-(3-[4-(6-Fluoro-1H-indazol-3-yl)-piperidin-1-yl]-propoxy)psoralen, dihydrochloride

Starting from 6-fluoro-3-(4-piperidinyl)-1H-indazole (270 mg; 1.23 mmol), 9-(3-bromopropoxy)psoralen (484.5 mg; 1.5 mmol) and potassium carbonate (500 mg; 2.66 mmol) using the procedure described in example 1 was prepared 150 mg (22.8%) of the title compound.

NMR DMSO-$d_6$ (ppm): 2.15 (br d, 2H), 2.32 (m, 5H), 3.18 (m, 2H), 3.37 (m, 2H), 3.62 (br. d, 2H), 4.5 (t, 2H), 6.45 (d, 1H), 6.98 (t, 1H), 7.15 (s, 1H), 7.30 (d, 1H), 7.78 (s, 1H), 7.98 (q, 1H), 8.15 (m, 2H), 10.9 (s, 1H), 13.0 (br., 1H).

**Claims**

**1.** A compound of the general formula I

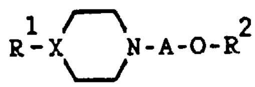

(I)

wherein A is a saturated or unsaturated, straight or branched hydrocarbon containing 2-6 carbon atoms;
R' is

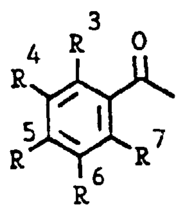

wherein $R^3$, $R^4$, $R^5$, $R^6$ and $R^7$ independently are hydrogen, halogen, $C_{1-6}$-alkyl, $C_{3-8}$-cycloalkyl, cyano, nitro or perhalomethyl; or $R^1$ is

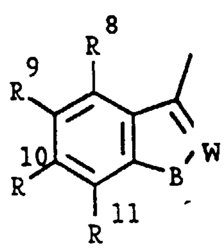

wherein $R^8$, $R^9$, $R^{10}$ and $R^{11}$ independently are hydrogen, halogen, $C_{1-6}$-alkyl, $C_{3-8}$-cycloalkyl, cyano, nitro or perhalomethyl; or
$R^1$ represents phenyl, pyridyl, pyrimidyl or pyrazinyl optionally mono- or disubstituted with $C_{1-6}$-alkoxy, halogen, cyano, nitro, acetyl or perhalomethyl, or
$R^1$ is 1-naphtyl, 2-naphtyl, azanaphtyl or diazanapthyl either of which may be substituted with $C_{1-6}$-alkyl, $C_{3-8}$-cycloalkyl, $C_{1-6}$-alkoxy, halogen, cyano, nitro, acetyl or perhalomethyl;

B is O, S or NH;

W is N or CH;

X is CH or N;

$R^2$ is a group

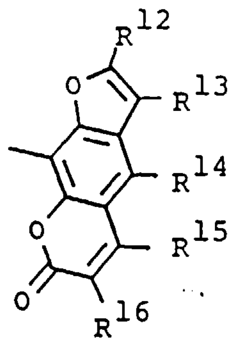
(II)
,
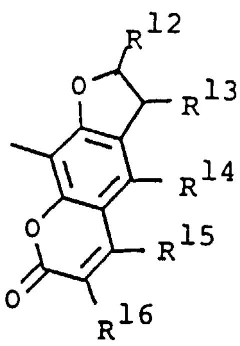
(III)
or
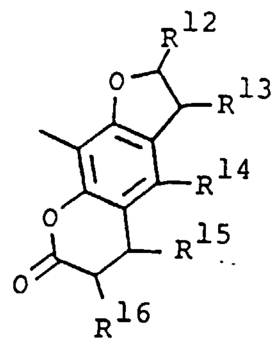
(IV)

wherein $R_{12}$, $R_{13}$, $R_{14}$, $R_{15}$ and $R_{16}$ independently are hydrogen, halogen, $C_{1-6}$-alkyl, cyano, nitro and perhalomethyl;

or pharmaceutically acceptable salts thereof.

**2.** A compound according to claim 1 which is

9-(3-(4-(2-Chlorophenyl)piperazin-1-yl)propoxy)psoralen, HCl
9-(3-(4-(1-Naphthyl)piperazin-1-yl)propoxy)psoralen, HCl
9-(3-(4-(6-Fluoro-1,2-benzisoxazol-3-yl)piperidino)propoxy) psoralen, oxalate
9-(3-(4-(2-Pyridylpiperazin-1-yl)propoxy)psoralen, HCl
9-(3-(4-(4-Fluorobenzoyl)piperidino)propoxy)psoralen, HCl
9-(3-(4-(4-Chlorophenyl)piperazin-1-yl)propoxy)psoralen, HCl
9-(3-(4-(4-Acetylphenyl) piperazin-1-yl) propoxy)psoralen, HCl
9-(3-(4-(3,4-Dichlorophenyl)piperazin-1-yl)propoxy)psoralen, HCl
9-(3-(4-(8-Quinolinyl)piperazin-1-yl)propoxy)psoralen, HCl
9-(4-(4-(4-Fluorobenzoyl)piperidino)butyloxy)psoralen, oxalate
9-(4-(4-(6-Fluoro-1,2-benzisoxazol-3-yl)piperidino)butyloxy)psoralen, oxalate
9-(4-(4-Fluorobenzoyl)piperidino)butyloxy)-6,7-dihydropsoralen, oxalate
9-(2-(4-(4-Fluorobenzoyl)piperidino)ethoxy)psoralen, oxalate
9-(2-(4-(6-Fluoro-1,2-benzisoxazol-3-yl)piperidino)ethoxy)psoralen, oxalate
9-(2-(4-(1,2-Benzisothiazol-3-yl)piperazin-1-yl)ethoxy)psoralen, oxalate
5-Chloro-9-(4-(4-(6-fluoro-1,2-benzisoxazol-3-yl)-1-piperidyl)butyloxy)psoralen, HCl
5-Bromo-9-(4-(6-fluoro-1,2-benzisoxazol-3-yl)-1-piperidyl)butyloxy)psoralen, HCl
9-(2-(4-(2-Pyrimidyl)piperazin-1-yl)ethoxy)psoralen, oxalate
9-(2-(4-(2-Pyridyl)piperazin-1-yl)-ethoxy)psoralen, HCl
9-(2-(4-(2-Chlorophenyl)piperazin-1-yl)ethoxy)psoralen, hydrochloride
9-{3-[4-(6-Fluoro-1H-indazol-3-yl)piperidin-1-yl]propoxy}psoralen, dihydrochloride.

**3.** A compound according to claim 1 or 2 or a pharmaceutically acceptable salt thereof for use as a therapeutically acceptable substance.

**4.** A compound according to claim 1 or 2 or a pharmaceutically acceptable salt thereof for use as a therapeutically acceptable substance in the treatment of CNS-system, cardiovascular system or gastrointestinal disorders.

**5.** A method of preparing compounds of formula I according to claim 1, which comprises

a) reacting a compound of formula (V)

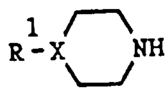

(V)

wherein $R^1$ and X have the meanings set forth above, with a compound of formula (VI)

$$Y\text{-}A\text{-}O\text{-}R^2 \quad (VI)$$

wherein A has the meaning set forth above and Y is a suitable leaving group, to form a compound of formula I, wherein $R^2$ is a group (II),

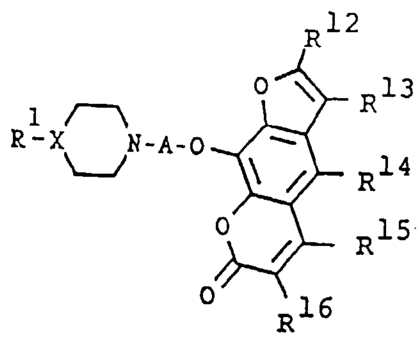

or

b) catalytical reduction of a compound of formula (I), wherein $R^2$ is a group (II)

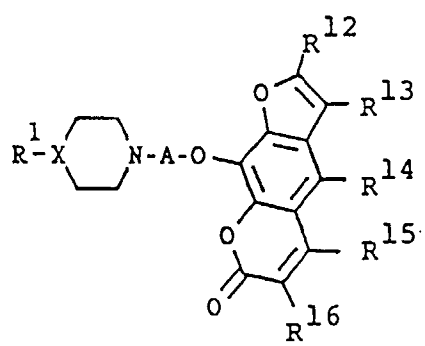

to form a compound of formula I, wherein $R^2$ is a group (III) or a group (IV)

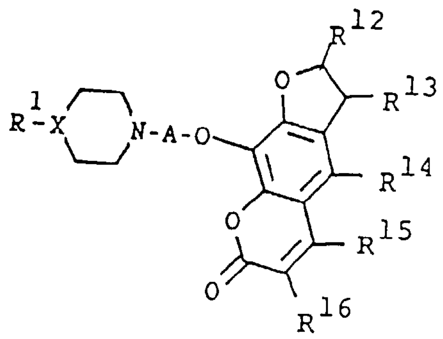

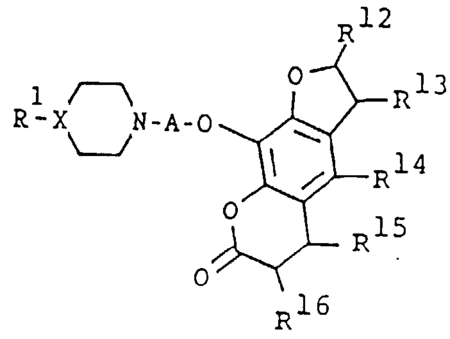

6. A pharmaceutical composition comprising a compound according to claim 1 or 2 or a pharmaceutically acceptable salt thereof and a therapeutically inert excipient.

7. A pharmaceutical composition suitable for the treatment of CNS-system, cardiovascular disorders or gastrointestinal disorders, which comprises a compound according to claim 1 or 2 or a pharmaceutically acceptable salt thereof and a therapeutically inert excipient, carrier or diluent.

8. Use of a compound of formula (I) according to claim 1 or 2 or a pharmaceutically acceptable salt thereof for the manufacture of a medicament for the treatment of CNS-system disorders, cardiovascular disorders or gastrointestinal disorders.

9. A process for the manufacture of a medicament, particularly to be used in the treatment of CNS-system disorders, cardiovascular disorders or gastrointestinal disorders which process comprises bringing a compound of formula (I) according to claim 1 or 2 or a pharmaceutically acceptable salt thereof into a galenical dosage form.

**Patentansprüche**

1. Eine Verbindung der allgemeinen Formel I

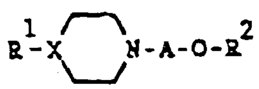

(I)

worin A ein gesättigter oder ungesättigter, gerader oder verzweigter Kohlenwasserstoff ist, der 2 - 6 Kohlenstoffatome enthält;
$R^1$

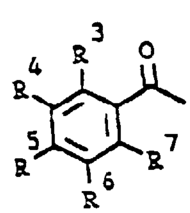

ist, worin $R^3$, $R^4$, $R^5$, $R^6$ und $R^7$ unabhängig Wasserstoff, Halogen, $C_{1-6}$-Alkyl, $C_{3-8}$-Cycloalkyl, Cyano, Nitro oder Perhalomethyl sind; oder $R^1$

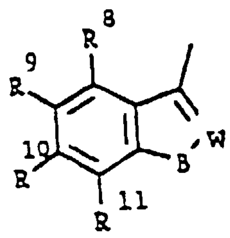

ist, worin $R^8$, $R^9$, $R^{10}$ und $R^{11}$ unabhängig Wasserstoff, Halogen, $C_{1-6}$-Alkyl, $C_{3-8}$-Cycloalkyl, Cyano, Nitro oder Perhalomethyl sind; oder

$R^1$ Phenyl, Pyridyl, Pyrimidyl oder Pyrazinyl darstellt, fakultativ mono- oder disubstituiert mit $C_{1-6}$-Alkoxy, Halogen, Cyano, Nitro, Acetyl oder Perhalomethyl, oder

$R^1$ 1-Naphthyl, 2-Naphthyl, Azanaphthyl oder Diazanaphthyl ist, von denen jede mit $C_{1-6}$-Alkyl, $C_{3-8}$-Cycloalkyl, $C_{1-6}$-Alkoxy, Halogen, Cyano, Nitro, Acetyl oder Perhalomethyl substituiert sein kann;

B O, S oder NH ist;

W N oder CH ist;

X CH oder N ist;

$R^2$ eine Gruppe

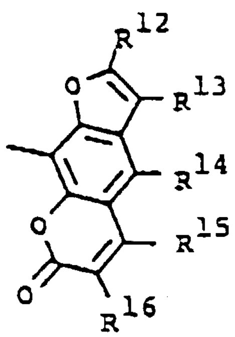

(II)

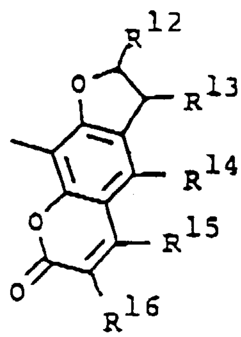

(III)

or

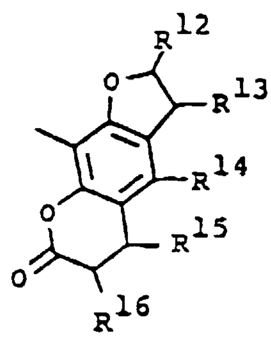

(IV)

ist, worin $R^{12}$, $R^{13}$, $R^{14}$, $R^{15}$ und $R^{16}$ unabhängig Wasserstoff, Halogen, $C_{1-6}$-Alkyl, Cyano, Nitro und Perhalomethyl sind;

oder pharmazeutisch annehmbare Salze derselben.

**2.** Eine Verbindung nach Anspruch 1, die

9-(3-(4-(2-Chlorphenyl)piperazin-1-yl)propoxy)psoralen, HCl
9-(3-(4-(1-Naphtyl)piperazin-1-yl)propoxy)psoralen, HCl
9-(3-(4-(6-Fluor-1,2-benzisoxazol-3-yl)piperidino)propoxy)psoralen, Oxalat
9-(3-(4-(2-Pyridylpiperazin-1-yl)propoxy)psoralen, HCl
9-(3-(4-(4-Fluorbenzoyl)piperidino)propoxy)psoralen, HCl
9-(3-(4-(4-Chlorphenyl)piperazin-1-yl)propoxy)psoralen, HCl
9-(3-(4-(4-Acetylphenyl)piperazin-1-yl)propoxy)psoralen, HCl
9-(3-(4-(3,4-Dichlorphenyl)piperazin-1-yl)propoxy)psoralen, HCl
9-(3-(4-(8-Chinolinyl)piperazin-1-yl)propoxy)psoralen, HCl

9-(4-(4-(4-Fluorbenzoyl)piperidino)butyloxy)psoralen,Oxalat
9-(4-(4-(6-Fluor-1,2-benzisoxazol-3-yl)piperidino)butyloxy) psoralen, Oxalat
9-(4-(4-Fluorbenzoyl)piperidino)butyloxy)-6,7-dihydropsoralen, Oxalat
9-(2-(4-(4-Fluorbenzoyl)piperidino)ethoxy)psoralen, Oxalat
9-(2-(4-(6-Fluor-1,2-benzisoxazol-3-yl)piperidino)ethoxy)psoralen, Oxalat
9-(2-(4-(1,2-Benzisothiazol-3-yl)piperazin-1-yl)ethoxy)psoralen, Oxalat
-5-Chlor-9-(4-(4-(6-fluor-1,2-benzisoxazol-3-yl)-1-piperidyl) -butyloxy)psoralen, HCl
5-Brom-9-(4-(6-fluor-1,2-benzisoxazol-3-yl)-1-piperidyl)butyloxy)psoralen, HCl
9-(2-(4-(2-Pyrimidyl)piperazin-1-yl)ethoxy)psoralen,Oxalat
9-(2-(4-(2-Pyridyl)piperazin-1-yl)-ethoxy)psoralen, HCl
9-(2-(4-(2-Chlorphenyl)piperazin-1-yl)ethoxy)psoralen, Hydrochlorid
9-{3-[4-(6-Fluor-1H-indazol-3-yl)piperidin-1-yl]propoxy}psoralen, Dihydrochlorid

ist.

**3.** Eine Verbindung nach Anspruch 1 oder 2 oder ein pharmazeutisch annehmbares Salz derselben zur Verwendung als eine therapeutisch annehmbare Substanz.

**4.** Eine Verbindung nach Anspruch 1 oder 2 oder ein pharmazeutisch annehmbares Salz derselben zur Verwendung als eine therapeutisch annehmbare Substanz bei der Behandlung des CNS-Systems, des Herz-Kreislauf-Systems oder von Magen-Darm-Störungen.

**5.** Ein Verfahren zur Herstellung von Verbindungen von Formel I nach Anspruch 1, welches umfaßt

a) Umsetzen einer Verbindung von Formel (V)

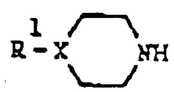

(V)

worin $R^1$ und X die oben angegebenen Bedeutungen haben, mit einer Verbindung von Formel (VI)

$$Y\text{-}A\text{-}O\text{-}R^2 \qquad (VI)$$

worin A die oben angegebene Bedeutung hat und Y eine geeignete Abgangsgruppe ist, um eine Verbindung von Formel I zu bilden, worin $R^2$ eine Gruppe <u>(II) ist</u>

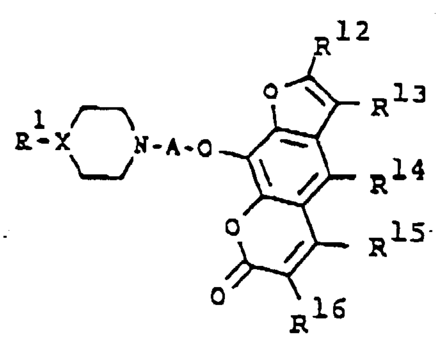

oder

b) katalytische Reduktion einer Verbindung von Formel (I), worin $R^2$ eine Gruppe (II) ist

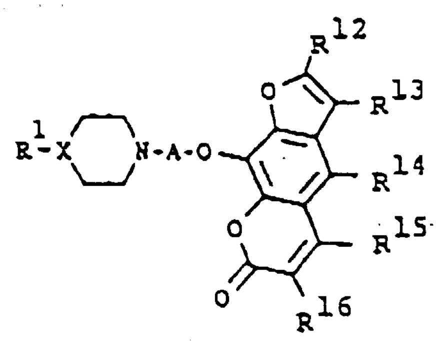

um eine Verbindung von Formel I zu bilden, worin $R^2$ eine Gruppe (III) oder eine Gruppe (IV) ist

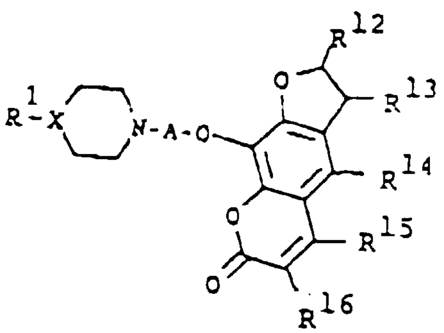

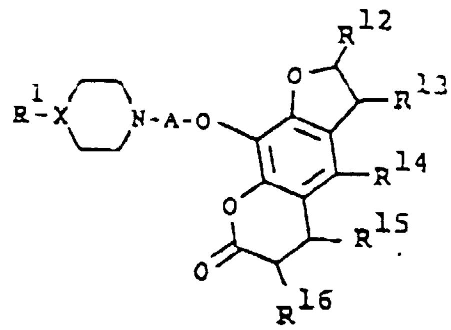

6. Eine pharmazeutische Zusammensetzung, die eine Verbindung nach Anspruch 1 oder 2 oder ein pharmazeutisch annehmbares Salz derselben und einen therapeutisch inerten Hilfsstoff umfaßt.

7. Eine pharmazeutische Zusammensetzung, die zur Behandlung des CNS-Systems, von Herz-Kreislauf-Störungen oder von Magen-Darm-Störungen geeignet ist, welche eine Verbindung nach Anspruch 1 oder 2 oder ein pharmazeutisch annehmbares Salz derselben und einen therapeutisch inerten Hilfsstoff, Trägerstoff oder Verdünnungsmittel umfaßt.

8. Verwendung einer Verbindung von Formel (I) nach Anspruch 1 oder 2 oder eines pharmazeutisch annehmbaren Salzes derselben zur Herstellung eines Arzneimittels zur Behandlung von Störungen des CNS-Systems, Herz-Kreislauf-Störungen oder Magen-Darm-Störungen.

9. Ein Verfahren zur Herstellung eines Arzneimittels, das insbesondere verwendet werden soll zur Behandlung von Störungen des CNS-Systems, Herz-Kreislauf-Störungen oder Magen-Darm-Störungen, wobei das Verfahren umfaßt, daß eine Verbindung von Formel (I) nach Anspruch 1 oder 2 oder ein pharmazeutisch annehmbares Salz derselben in eine galenische Dosierungsform gebracht wird.

**Revendications**

1. Composé de formule générale (I)

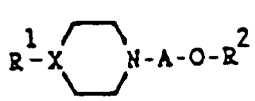

(I)

où A est un hydrocarbure saturé ou insaturé, linéaire ou ramifié, contenant 2 - 6 atomes de carbone;
$R^1$ est

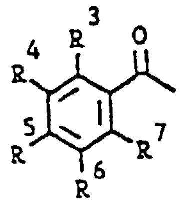

où $R^3$, $R^4$, $R^5$, $R^6$ et $R^7$ sont indépendamment hydrogène, halogène, alkyle en $C_{1-6}$, cycloalkyle en $C_{3-8}$, cyano, nitro ou perhalométhyle, ou $R^1$ est

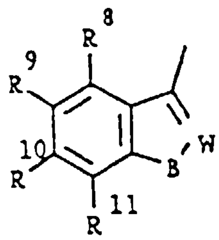

où $R^8$, $R^9$, $R^{10}$ et $R^{11}$ sont indépendamment hydrogène, halogène, alkyle en $C_{1-6}$, cycloalkyle en $C_{3-8}$, cyano, nitro ou perhalométhyle; ou

$R^1$ représente phényle, pyridyle, pyrimidyle ou pyrazinyle éventuellement mono- ou disubstitué par alkoxy en $C_{1-6}$, halogène, cyano, nitro, acétyle ou perhalométhyle, ou $R^1$ est 1-naphtyle, 2-naphtyle, azanaphtyle ou diazanaphtyle dont l'un ou l'autre peut être substitué par alkyle en $C_{1-6}$, cycloalkyle en $C_{3-8}$, alkoxy en $C_{1-6}$, halogène, cyano, nitro, acétyle ou perhalométhyle;

B est O, S ou NH;

W est N ou CH;

X est CH ou N;

$R^2$ est un groupe

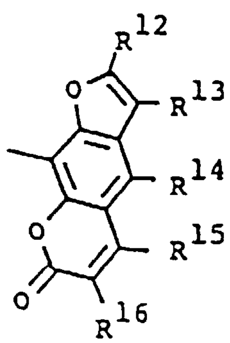

(II)

,

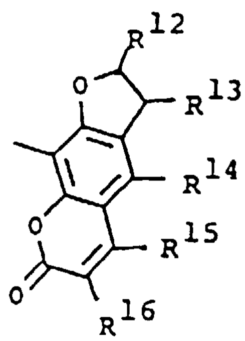

(III)

ou

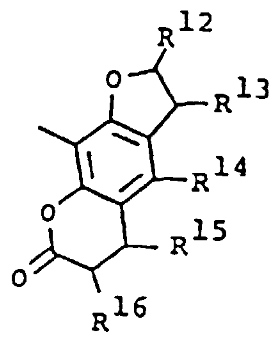

(IV)

où $R^{12}$, $R^{13}$, $R^{14}$, $R^{15}$ et $R^{16}$ sont indépendamment hydrogène, halogène, alkyle en $C_{1-6}$, cyano, nitro et perhalométhyle; ou sels pharmaceutiquement acceptables de celui-ci.

**2.** Composé selon la revendication 1 qui est

le 9-(3-(4-(2-chlorophényl)pipérazin-1-yl)propoxy)psoralène, HCl
le 9-(3-(4-(1-naphthyl)pipérazin-1-yl)propoxy)psoralène, HCl
le 9-(3-(4-(6-fluoro-1,2-benzisoxazol-3-yl)pipéridino)propoxy)psoralène, oxalate
le 9-(3-(4-(2-pyridylpipérazin-1-yl)propoxy)psoralène,HCl
le 9-(3-(4-(4-fluorobenzoyl)pipéridino)propoxy)psoralène, HCl

le 9-(3-(4-(4-chlorophényl)pipérazin-1-yl)propoxy)psoralène, HCl
le 9-(3-(4-(4-acétylphényl)pipérazin-1-yl)propoxy)psoralène, HCl
le 9-(3-(4-(3,4-dichlorophényl)pipérazin-1-yl)propoxy)psoralène, HCl
le 9-(3-(4-(8-quinolinyl)pipérazin-1-yl)propoxy)psoralène, HCl
le 9-(4-(4-(4-fluorobenzoyl)pipéridino)butyloxy)psoralène, oxalate
le 9-(4-(4-(6-fluoro-1,2-benzisoxazol-3-yl)pipéridino)butyloxy)psoralène, oxalate
le 9-(4-(4-fluorobenzoyl)pipéridino)butyloxy)-6,7-dihydropsoralène, oxalate
le 9-(2-(4-(4-fluorobenzoyl)pipéridino)éthoxy)psoralène, oxalate
le 9-(2-(4-(6-fluoro-1,2-benzisoxazol-3-yl)pipéridino)éthoxy)psoralène, oxalate
le 9-(2-(4-(1,2-benzisothiazol-3-yl)pipérazin-1-yl)éthoxy)psoralène, oxalate
le 5-chloro-9-(4-(4-(6-fluoro 1,2-benzisoxazol-3-yl)-1-pipéridyl)butyloxy)psoralène, HCl
le 5-bromo-9-(4-(6-fluoro-1,2-benzisoxazol-3-yl)-1-pipéridyl)butyloxy)psoralène, HCl
le 9-(2-(4-(2-pyrimidyl)pipérazin-1-yl)éthoxy)psoralène, oxalate
le 9-(2-(4-(2-pyridyl)pipérazin-1-yl)éthoxy)psoralène, HCl
le 9-(2-(4-(2-chlorophényl)pipérazin-1-yl)éthoxy)psoralène, chlorhydrate
le 9-{3-[4-(6-fluoro-1H-indazol-3-yl)pipéridin-1-yl]propoxy}psoralène, dichlorhydrate.

**3.** Composé selon la revendication 1 ou 2 ou sel pharmaceutiquement acceptable de celui-ci destiné à être utilisé comme substance thérapeutiquement acceptable.

**4.** Composé selon la revendication 1 ou 2 ou sel pharmaceutiquement acceptable de celui-ci destiné à être utilisé comme substance thérapeutiquement acceptable dans le traitement de troubles du système SNC, du système cardio-vasculaire ou gastro-intestinaux.

**5.** Procédé de préparation de composés de formule (I) selon la revendication 1, qui comprend

a) la réaction d'un composé de formule (V)

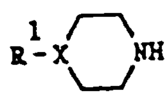

(V)

où $R^1$ et X ont les significations énoncées ci-dessus, avec un composé de formule (VI)

$$Y\text{-}A\text{-}O\text{-}R^2 \quad (VI)$$

où A a la signification énoncée ci-dessus et Y est un groupe partant approprié, pour former un composé de formule (I) où $R^2$ est un groupe (II)

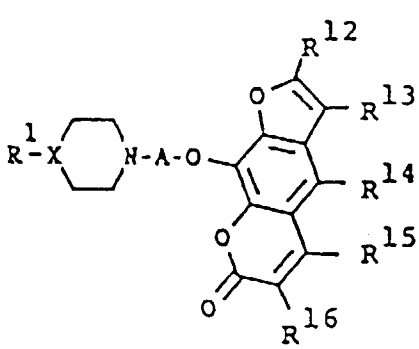

ou

b) la réduction catalytique d'un composé de formule (I) où $R^2$ est un groupe (II)

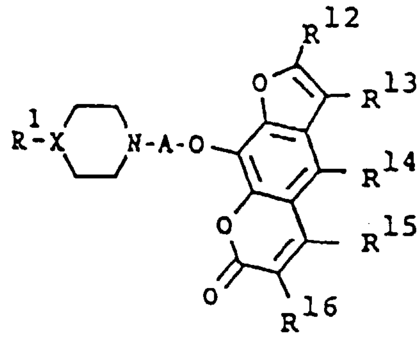

pour former un composé de formule (I) où $R^2$ est un groupe (III) ou un groupe (IV)

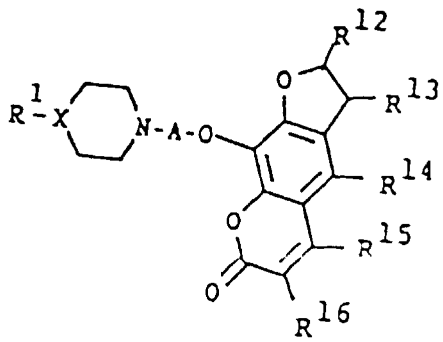

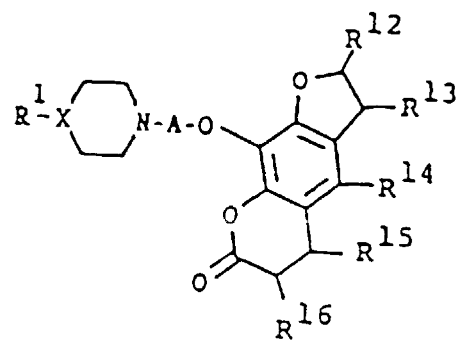

6. Composition pharmaceutique comprenant un composé selon la revendication 1 ou 2 ou un sel pharmaceutiquement acceptable de celui-ci et un excipient thérapeutiquement inerte.

7. Composition pharmaceutique appropriée au traitement de troubles du système SNC, de troubles cardio-vasculaires ou de troubles gastro-intestinaux, qui comprend un composé selon la revendication 1 ou 2 ou un sel pharmaceutiquement acceptable de celui-ci et un excipient, support ou diluant thérapeutiquement inerte.

8. Utilisation d'un composé de formule (I) selon la revendication 1 ou 2 ou d'un sel pharmaceutiquement acceptable de celui-ci pour la fabrication d'un médicament pour le traitement de troubles du système SNC, de troubles cardio-vasculaires ou de troubles gastro-intestinaux.

9. Procédé de fabrication d'un médicament, destiné en particulier à être utilisé dans le traitement de troubles du système SNC, de troubles cardio-vasculaires ou de troubles gastro-intestinaux, lequel procédé comprend l'incorporation d'un composé de formule (I) selon la revendication 1 ou 2 ou d'un sel pharmaceutiquement acceptable de celui-ci dans une forme galénique.